(19) Europäisches Patentamt
European Patent Office
Office européen des brevets

(11) Veröffentlichungsnummer: **0 403 751 B1**

(12) # EUROPÄISCHE PATENTSCHRIFT

(45) Veröffentlichungstag der Patentschrift: **23.03.94**

(51) Int. Cl.$^5$: **C07D 233/02**, C11D 1/08

(21) Anmeldenummer: **90107051.6**

(22) Anmeldetag: **12.04.90**

Die Akte enthält technische Angaben, die nach dem Eingang der Anmeldung eingereicht wurden und die nicht in dieser Patentschrift enthalten sind.

(54) **Polycarbonsäureimidazoline, Verfahren zu ihrer Herstellung und ihre Verwendung in Reinigungsmitteln.**

(30) Priorität: **19.06.89 DE 3919863**

(43) Veröffentlichungstag der Anmeldung:
**27.12.90 Patentblatt 90/52**

(45) Bekanntmachung des Hinweises auf die Patenterteilung:
**23.03.94 Patentblatt 94/12**

(84) Benannte Vertragsstaaten:
**AT BE CH DE DK ES FR GB GR IT LI LU NL SE**

(56) Entgegenhaltungen:
**DE-A- 3 137 044**

**H. Hitz et al , Parfumerie und Kosmetik, 64 (1983), S. 16-21**

(73) Patentinhaber: **CHEMISCHES INSTITUT SCHAEFER AG**
**Hauptstrasse 159**
**CH-4416 Bubendorf(CH)**

Patentinhaber: **Joh. A. Benckiser GmbH**
**Postfach 21 10 67,**
**Ludwig-Bertram-Strasse 8 + 10**
**D-67059 Ludwigshafen(DE)**

(72) Erfinder: **Hitz, Hans, Dr.**
**Blauen-Rain-Strasse 25**
**CH-4422 Arisdorf(CH)**
Erfinder: **Schäfer, Rolf, Dr.**
**Im Rüggli 12**
**CH-4422 Arisdorf(CH)**
Erfinder: **Baust, Heinrich**
**Westende 29**
**D-6831 Plankstadt(DE)**
Erfinder: **Gross, Wolfgang, Dr.**
**Ludwigshafener Strasse 20**
**D-6704 Mutterstadt(DE)**

(74) Vertreter: **Grussdorf, Jürgen, Dr. et al**
**Patentanwälte Zellentin & Partner**
**Rubensstrasse 30**
**D-67061 Ludwigshafen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Reinigungsmittel, insbesondere Körper- und Haarwaschmittel, sowie Textil- und Geschirrwaschmittel, aber auch Industriereiniger sind im Allgemeinen Abmischungen von Tensiden und Tensidhilfsmitteln. Die hierfür verwendeten Tenside, sowie ihre Abmischungen untereinander, bestehen aus anionischen, neutralen, amphoteren und/oder kationischen oberflächenaktiven Stoffen. Diese oberflächenaktiven Stoffe enthalten einen hydrophoben Teil, welcher aus Kohlenwasserstoffen besteht, sowie einen hydrophilen oder polaren Teil.

Bei den anionischen Tensiden besteht nun der hydrophile Teil aus einer negativ geladenen Gruppierung, wobei es sich um Sulfate, Sulfonate, Phosphate, Phosphonate oder Carboxylate handelt. Diese Gruppierungen können auch über Polyoxiethylketten mit den Kohlenwasserstoffketten verknüpft sein. Während die Sulfonat- und Sulfat-haltigen Kohlenwasserstoffe stark anionisch geladene Tenside repräsentieren, so sind die Phosphate, Phosphonate und Carboxylate schwach anionische Tenside.

Bei den neutralen Tensiden besteht der hydrophile Teil aus einer polaren, neutralen Gruppierung, z. B. aus Aminoxyden, Polyoxethylresten, Kohlehydraten oder polyoxiethylierten Kohlehydraten.

Bei den amphoteren Tensiden handelt es sich bei der polaren Gruppe um Zwitterionen, das heisst diese Gruppen enthalten sowohl positive als auch negative Ladungen. Hierunter fallen insbesondere die Betaine, die Eiweisskondensate, sowie die Taurate.

Bei den kationischen Tensiden besteht die polare Gruppe aus mono-, di-, tri- und quartär substituierten Aminen, welche allesamt im neutralen pH-Bereich positiv geladen sind.
Währenddem die mono-, di-, und tri- substituierten Amine als schwach kationische Tenside bezeichnet werden, so sind die quartär substituierten Amine stark kationisch.

Die oben beschriebenen Tenside und deren Abmischungen sind problematisch in Bezug auf biologische Systeme:
Bei Tensidabmischungen im Körperpflegebereich kommt es zum Kontakt zwischen den Formulierungen und der Oberfläche der Haut. Solche tensidhaltigen Abmischungen können stärker oder weniger stark an der Oberfläche der Haut binden und die mikrobielle Hautflora - das natürliche Immunitätssystem der Haut - negativ beeinflussen.

Die Wechselwirkung der Tenside mit der mikrobiellen Hautflora kann zu deren Veränderung führen, sie schwächen, und ist als Folge Angriffspunkt für Infektionen fremder Mikroben und Pilze. Als Resultat davon können sich Allergien und Dermatosen manifestieren, eine Tatsache, welche heutzutage zunehmend beobachtet wird.

Bei der Wechselwirkung der Tenside mit der Haut kommt es in vielen Fällen zu Austrocknung, Abschuppungen, und nach chronischer Wechselwirkung zu Mikroläsionen.

Nach der Reinigung von Textilien sowie festen Oberflächen, welche tensidhaltige Abwässer hinterlassen, kommt es zwischen den Tensiden und den Mikroorganismen der Abwasseraufbereitungsanlagen zu einer Wechselwirkung, wobei diese Tenside die Lebensfähigkeit der stoffabbauenden Mikroflora beeinträchtigen können oder diese abtöten können.

Tenside verhalten sich somit oft als echte Zellgifte. Dies trifft für die stark anionischen und insbesondere für die stark kationischen Tenside zu.

Es wurde deshalb versucht durch spezielle Abmischungen der beschriebenen Tenside untereinander Formulierungen zu erhalten, welche die Toxizität respektive die Agressivität der stark anionischen sowie der stark kationischen Tenside herabsetzen, das heisst detoxifizieren.

Dies wurde durch die Wahl günstiger Formulierungsverhältnisse von neutralen und amphoteren Tensiden mit anionischen Tensiden erreicht. Die anionischen Tenside, speziell die Alkylsulfate und die Alkylethersulfate sind die in den heutigen Reinigungs- und Pflegemitteln meistverwendeten oberflächenaktiven Stoffe.

Diese Detoxifizierung ist allerdings relativ, das heisst, dass die stark anionischen und stark kationischen Tenside nur bis zu einem bestimmten Prozentsatz detoxifiert werden können.

Bei solchen relativ detoxifizierten Tensidmischungen spricht man von milden Formulierungen.

Die Restaggressivität der dem Stand der Technik beschriebenen milden Tensidformulierungen manifestiert sich auch dadurch, dass bei Kontakt dieser Stoffe mit der Cornea und der Mukosa im Auge im Normalfall ein starkes Brennen auftaucht, welches bis zur echten Irritation des Auges gehen kann.

In letzter Zeit ist zusätzlich auf die Spurenproblematik solcher tensidhaltigen Abmischungen hingewiesen worden:
bei den oxethylierten Produkten treten als Synthesenebenprodukte die Dioxane, bei den Aminen die Nitrosamine und bei den Sulfaten die Sultone auf, welche allesammt hoch toxisch sind respektive kanzerogenes Potential besitzen.

Aus H. Hitz et al Parfümerie und Kosmetik, 64 (1983) S. 16-21 ist eine neue Gruppe von 2-Alkylimidazolin-N-propionsäuren (Amphonyle) bekannt geworden, welche mildere Tenside darstellen als andere Amphotere. Diese geben allerdings erhebliche Formulierungsprobleme auf, da sie substantiell sehr schlecht viskositätsaufbauend sind und im alkalischen Medium unter Aufspaltung des Imidazolringes hydrolysieren.

Es stellte sich daher die Aufgabe, neue stark wirksame Tenside zu finden, die allein oder in Mischung mit anderen Tensiden keine oder nur geringe Agressivität, insbesondere Haut- und Augenirritation, besitzen, biologisch abbaubar sind und auch im sauren und alkalischen Milieu stabil sind.

Diese Aufgabe wird durch die in den Ansprüchen näher gekennzeichneten Verbindungen gelöst.

Gegenstand der Erfindung sind demgemäss Polycarbonsäureimidazoline der allgemeinen Formel I

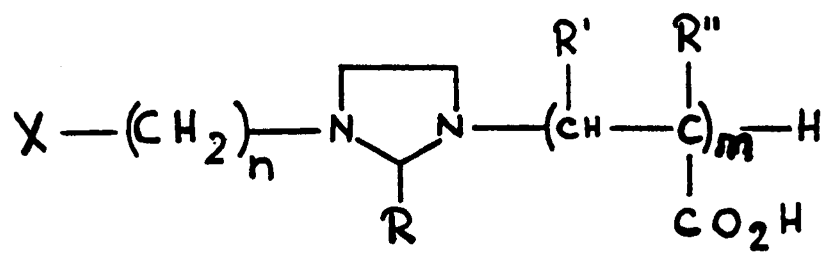

(I)

wobei X = -Cl, -OH, $-OSO_3H$, $-OPO_3H_2$, $-O-CH_2-CH_2COOH$, $-NH_2$, -NHalk, $N(alk)_2$, mit alk = nieder Alkyl mit 1 - 6 C-Atomen, bedeutet,
n eine ganze Zahl von 2-4,
R einen Kohlenwasserstoffrest der Kettenlänge $C_8$-$C_{18}$ repräsentiert welcher geradkettig, verzweigt, gesättigt oder 1 bis 3fach ungesättigt ist,
m eine ganze Zahl von 2 bis 5 bedeutet,
R′ ein Wasserstoffatom oder eine Carboxylgruppe und
R″ ein Wasserstoffatom oder eine Methylgruppe bedeutet.

Zur Herstellung der Verbindungen der Formel I wird von den bekannten Alkylimidazolin-Verbindungen der Formel II

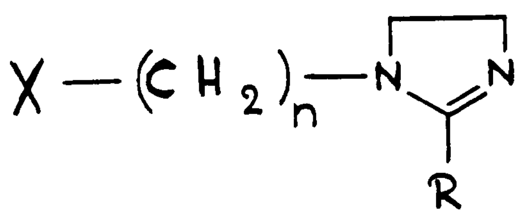

(II)

in der R, X und n die obige Bedeutung haben
ausgegangen und diese mit Acrylsäure, Methacrylsäure, Maleinsäure oder aktiven Derivaten oder Mischungen davon in einem 2 bis 5fachen Ueberschuss kondensiert.

Ueberraschenderweise wird bei einem grösseren Ueberschuss der ungesättigten Carbonsäure eine Polymerisation in der Carbonsäureseitenkette beobachtet, während nach der Vorliteratur (s.o.) eine Derivatisierung des Substituenten X zu erwarten war.

Die Kondensation wird vorzugsweise in der Schmelze der Komponenten bei Temperaturen von 100-150° C durchgeführt, jedoch kann zur Erleichterung der Durchmischung auch ein inertes Lösungsmittel wie Toluol oder Xylol oder ein hochsiedendes Paraffin zugesetzt werden.

Die Reaktion führt dabei nicht zu einem einheitlichen Produkt, sondern zu einem Gemisch von Verbindungen mit verschiedenen Kettenlängen m, wobei je nach Ueberschuss der Carbonsäure m bei 2-4 ein Maximum erreicht.

Eine chromatographische Trennung der Reaktionskomponenten ist möglich (beispielsweise mit Anionenaustauschern), jedoch für den Einsatz der Verbindungen als Tenside nicht erforderlich. Aus wirtschaftlichen Gründen wird daher bevorzugt das entstandene Gemisch direkt verwendet.

Die Ausgangsprodukte der Formel II sind bekannt oder lassen sich in bekannter Weise durch Kondensation von Verbindungen der Formel III

$X-(CH_2)_n-NH-CH_2-CH_2-NH_2$ (III)

mit Fettsäuren oder Fettsäureester der Formel IV

$R-CO_2-Y$ (IV)

in der R die obige Bedeutung hat und Y Wasserstoff oder eine Akylgruppe bedeutet, unter Wasser- bzw. Alkoholabspaltung erhalten. Vorzugsweise werden die auch natürlich vorkommenden Triglyceride eingesetzt.

Bei der Verwendung dieser neuen Polycarbonsäureimidazoline allein oder in Gemisch mit anderen Tensiden wurde nun überraschenderweise gefunden, dass es möglich wurde, pH-stabile, mildeste Tensidformulierungen zu erstellen.

Im Bereich der Körperpflegemittel konnten ultramilde Formulierungen unter Verwendung der Verbindungen der Formel I erstellt werden, wobei Sulfate, Ethersulfate, Alkylsulfate und Alkylsulfonate wegen ihrer übertrieben hohen Toxizität weggelassen wurden. Hierbei konnte gleichzeitig die Spurenproblematik eliminiert werden.

Insbesondere wurden die erzielten Eigenschaften dieser ultramildesten Tensidformulierungen für den gesamten Körperpflegemittel- und Reinigungsmittelbereich an biologischen Modellen sowie am Menschen getestet und bestätigt.

Bei den biologischen Testmodellen handelt es sich um:

1. die Augen- und Augenschleimhautverträglichkeit
2. den Comforttest am menschlichen Auge
3. die dermale Verträglichkeit am Kaninchen
4. den Duringkammertest am Menschen
5. die Inhibition proteolytischer Aktivität mittels Trypsin
6. die kontaktallergenen Eigenschaften am Meerschweinchen
7. den Nutritionstest, welcher die Kompatibilität der menschlichen Hautflora mit den Formulierungen beschreibt.

Beim Nutritionstest handelt es sich um ein Verfahren, in welchem die primäre und sekundäre Toxizität an Mikroorganismen eines zu testenden Stoffes oder Stoffgemisches (im hiesigen Falle Tensidformulierungen) bestimmt werden. Im Falle von Körperpflegemitteln dienen als Mikroorganismen die verschiedenen, natürlichen Mikroben, welche auf der menschlichen Haut angesiedelt sind. Im Falle von Tensidgemischen in Abwässern dient die Mikroflora der Abwasseraufbereitungsanlagen als biologisches Modell.

Im Test werden nun die oben erwähnten Bakterien in einem kohlenstoffreien Medium suspendiert. Als Kohlenstoffquelle wird danach die zu testende Substanz (in diesem Fall Tenside oder Tensidformulierungen) in die Suspension gegeben. Anhand der Wachstumskurven der Mikroben lässt sich die primäre und sekundäre Toxizität des zu testenden Stoffes exakt bestimmen.

Die im Beispiel 11 angeführte ultramilde Formulierung eines Duschgels hat im Vergleich zu auf dem Markt befindlichen, typischen, sogenannt milden Babyshampoos (Stand der Technik) zu folgenden Resultaten geführt:

| | Formulierung (Beispiel 11) Irritations-Index | Babyshampoo (Stand der Technik) Irritations-Index |
|---|---|---|
| Augen- und Schleimhautverträglichkeit (Draize, Formulierung 1 : 5 mit $H_2O$ verdünnt, Augen ungespült) | 0.00 | 2.17 |
| Comfortest am menschlichen Auge (Formulierung 1 : 200 verdünnt) | 1.1 | 2.6 |
| Dermale Verträglichkeit am Kaninchen (Formulierung 1 : 5 verdünnt) | 1.7 | 2.7 |
| Duringkammer Test am Menschen (Formulierung 1 : 2.5 verdünnt) | 5 | 26 |
| Inhibition der proteolyt. Aktivität von Trypsin (Schwellkonzentration der WAS in Prozenten) | 7.5% | 4% |

| | | |
|---|---|---|
| Nutritionstest (Formulierung 1 : 20 verdünnt) Zellteilunsrate der humanen Hautflora (10% Wachstum in h) | 47 h | 172 h |

Aus den obenbeschriebenen Werten ist ersichtlich, dass die Formulierung der hier beschriebenen Erfindung in allen biologischen Modellen im Vergleich zum Stand der Technik als ultramild bezeichnet werden kann, wenn man davon ausgeht, dass es sich beim Stand der Technik um eine milde Formulierung handelt.

3. Beispiele

Beispiel 1

Aminoethylethanolamin (1.2 Mole) werden mit Maiskeimöl (1/3 Mol Triglycerid) bei 190 Grad Celsius für 2 Stunden unter Normaldruck gerührt. Anschliessend wird der Druck auf 10 mbar gesenkt und dabei über 2 Stunden das überschüssige Aminoethylethanolamin (20%) und das entstandene Glycerin destillativ aus dem Reaktionsgemisch entfernt. Die im Reaktionsgefäss entstandene Alkylimidazoliniumbase (1 Mol) wird auf 140 Grad Celsius gekühlt. Bei dieser Temperatur werden über 2 Stunden Acrylsäure (2.2 Mole) zur Imidazoliniumbase addiert und bei gleicher Temperatur über zwei weitere Stunden gerührt. Danach wird das Reaktionsprodukt auf 90 Grad Celsius abgekühlt und mit 1.5 Gewichtsteilen Wasser verdünnt und mit 10N NaOH auf pH 5 titriert (R = $C_{18:1}$-$C_{18:3}$, n = 2, m = 2-5; R′, R″ = H; X = -OH).
(ungefähre Zusammensetzung)
2 % Imidazol
5 % Imidazolpropionsäure
82 % Imidazoldiacrylsäure
8 % Imidazoltriacrylsäure
3 % Polyacrylsäure

Beispiel 2

Siehe Beispiel 1, jedoch wird Kokosöl anstelle von Maiskeimöl verwendet (R = $C_{14}$, n = 2, m = 2-5; R′, R″ = H; X = -OH).

Beispiel 3

Siehe Beispiel 1, jedoch wird Kokosvorlauffettsäuretriglycerid anstelle von Maiskeimöl verwendet (R = $C_8$-$C_{10}$; X = -OH).

Beispiel 4

Siehe Beispiel 1, jedoch wird Chlorethyl-ethylendiamin anstelle von Aminoethylethanolamin verwendet (n = 2, X = Cl).

Beispiel 5

Siehe Beispiel 1, jedoch wird Sulfatidyl-ethyl-ethylendiamin anstelle von Aminoethylethanolamin verwendet (n = 2, X = $OSO_3H$).

Beispiel 6

Siehe Beispiel 1, jedoch wird Phosphatidyl-ethyl-ethylendiamin anstelle von Aminoethylethanolamin verwendet (n = 2, X = $OPO_3H_2$).

Beispiel 7

Siehe Beispiel 1, jedoch wird Ethylendiamino-ethoxy-propionsäure anstelle von Aminoethylethanolamin verwendet (n = 2, X = $O\text{-}CH_2CH_2\text{-}CO_2H$).

Beispiel 8

Siehe Beispiel 1, jedoch wird Diethylentriamin-monohydrochlorid anstelle von Aminoethylethanolamin verwendet.

Beispiel 9

Siehe Beispiel 1, jedoch wird Oleyl-amino-ethyl-ethylendiamin anstelle von Aminoethylethanolamin verwendet.

Beispiel 10

Siehe Beispiel 1, jedoch wird Distearyl-amino-ethyl-ethylendiamin anstelle von Aminoethylethanolamin verwendet.

Beispiel 11

Duschgel:

| | | | |
|---|---|---|---|
| Gemisch gemäss Beispiel 2 | (40%) | 7.5 | Teile |
| Cocoylsulfosuccinat | (30%) | 15 | " |
| Cocoamidopropylbetain | (30%) | 20 | " |
| Stearylmonoglycerid | (20%) | 1 | " |
| Eiweisshydrolysat | (100%) | 0.2 | " |
| Lauryalkoholdecaethoxylat | (100%) | 0.2 | " |
| $H_2O$ ad | | 100 | Teile |

Beispiel 12

Conditionier-Shampoo/Babyshampoo:

| | | | |
|---|---|---|---|
| Gemisch gemäss Beispiel 1 | (40%) | 7.5 | Teile |
| Cocoylsulfosuccinat | (30%) | 15 | " |
| Cocoamidopropylbetain | (30%) | 20 | " |
| Stearylmonoglycerid | (20%) | 1 | " |
| Eiweisshydrolysat | (100%) | 0.2 | " |
| Lauryalkoholdecaethoxylat | (100%) | 0.2 | " |
| $H_2O$ ad | | 100 | Teile |

Beispiel 13

Schaumbad:

| | | | |
|---|---|---|---|
| Gemisch gemäss Beispiel 2 | (40%) | 7.5 | Teile |
| Cocoylsulfosuccinat | (30%) | 15 | " |
| Cocoamidopropylbetain | (30%) | 20 | " |
| Laurylmonoglycerid | (20%) | 1 | " |
| Eiweisshydrolysat | (100%) | 0.2 | " |
| Lauryalkoholdecaethoxylat | (100%) | 0.2 | " |
| Kamillenöl | (100%) | 2 | " |
| $H_2O$ ad | | 100 | Teile |

Beispiel 14

Gesichtswaschlotion:

| | | | |
|---|---|---|---|
| Gemisch gemäss Beispiel 2 | (40%) | 27.5 | Teile |
| Cocoylsulfosuccinat | (30%) | 15 | " |
| Stearylmonoglycerid | (20%) | 1 | " |
| Eiweisshydrolysat | (100%) | 0.2 | " |
| Laurylalkoholdecaethoxylat | (100%) | 0.2 | " |
| $H_2O$ ad | | 100 | Teile |

Beispiel 15

Cremebad/Oelbad:

| | |
|---|---|
| Gemisch gemäss Beispiel 2 | 10 Teile |
| Laurylalkoholoctaethoxylat | 10 Teile |
| Laurylalkoholdecaoxiethylcarboxylat | 5 " |
| Oleylalkohol | 20 " |
| Weizenkeimöl | 20 " |
| Maiskeimöl | 27 " |
| Lanolinalkohol | 8 " |

Beispiel 16

Pflegeemulsion:

| | |
|---|---|
| Gemisch gemäss Beispiel 1 | 3 Teile |
| Vaselin Oel | 6 Teile |
| PEG-20-Oleat | 5 Teile |
| Propylenglycol | 5.5 Teile |
| Collagen (10%) | 2 Teile |
| Hypericum-Extrakte | 0.9 Teile |
| $H_2O$ ad | 100 Teile |

Beispiel 17-19

Seifen:

a) Flüssigseife:

| | |
|---|---|
| Gemisch gemäss Beispiel 2 | 8 Teile |
| Ethoxyliertes Glycerylmonolaurat | 1 Teil |
| Lauryläthersulfat (28%) | 40 Teile |
| $H_2O$ ad | 100 Teile |

b) Stückseifen:

Naturseife:

| | |
|---|---|
| Gemisch gemäss Beispiel 2 | 5.5 Teile |
| Seifengrundmasse, 80% Gesamtfettsäuren-Na-Salz, Talg/Cocos 80:20 | 90 " |
| Polyphosphate | 3 " |
| Titandioxyd | 0.25 " |

Syndetseife:

| | |
|---|---|
| Gemisch gemäss Beispiel 2 | 9 Teile |
| Fettalkoholsulfat $C_{12}$-$C_{18}$-Na-Salz | 30 " |
| Polyphosphate | 3 " |
| Fettalkoholsulfosuccinat, Di-Na-Salz | 20 " |
| Stärke | 18 " |
| Paraffin | 9 " |
| Stearylalkohol | 6 " |
| Cetylalkohol | 4 " |

Beispiel 20

Handspülmittel:

| | |
|---|---|
| Gemisch gemäss Beispiel 1 | 5 Teile |
| Lineares Laurylbenzolsulfonat (100%) | 20 Teile |
| Eiweisshydrolysat | 0.5 Teile |
| $H_2O$ ad | 100 Teile |

Beispiel 21

Maschinengeschirrspülmittel (phosphatfrei):

| | |
|---|---|
| Gemisch gemäss Beispiel 3 | 5 Teile |
| Stearylalkoholpentaethoxylat | 10 Teile |
| Lauryltrioxyethylcarboxylat (50%) | 3 Teile |
| Natriummetasilikat/NaOH | 5 Teile |
| $H_2O$ ad | 100 Teile |

Beispiel 22

Feinwaschmittel/Wollwaschmittel:

| | |
|---|---|
| Gemisch gemäss Beispiel 2 | 4 Teile |
| Laurylbenzolsulfonat (100%) | 15 Teile |
| Lanolinalkohol | 15 Teile |
| $H_2O$ ad | 100 Teile |

Beispiel 23

Allzweckreiniger:

| | |
|---|---|
| Gemisch gemäss Beispiel 3 | 5 Teile |
| Sekundäres Alkansulfonat (100%) | 15 Teile |
| Kaliumoleat (100%) | 0.5 Teile |
| Natriumcarbonat | 2 Teile |
| Trinatriumcitrat | 1 Teil |
| $H_2O$ ad | 100 Teile |

Beispiel 24

Industriereiniger:

| | |
|---|---|
| Gemisch gemäss Beispiel 3 | 5 Teile |
| Laurylbenzolsulfonat | 10 Teile |
| Lauryläthersulfat (28%) | 5 Teile |
| $H_2O$ ad | 100 Teile |

## Patentansprüche

1. Polycarbonsäureimidazoline der allgemeinen Formel I

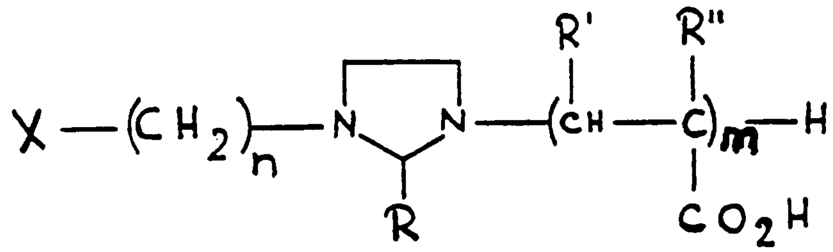

(I)

in der

X = -Cl, -OH, $-O-SO_3H$, $-O-PO_3H_2$, $-O-CH_2-CH_2-CO_2H$, $NH_2$, NHalk, $N(alk)_2$ mit alk = nieder Alkyl mit 1-6 C-Atomen,
n = eine ganze Zahl von 2-4,
R = ein geradkettiger, verzweigter, gesättigter oder 1-3fach ungesättigter Kohlenwasserstoffrest mit 8-18 C-Atomen,
m = eine ganze Zahl von 2 bis 5,
R′ = ein Wasserstoffatom oder eine Carboxylgruppe,
R″ = ein Wasserstoffatom oder eine Methylgruppe bedeuten.

2. Verfahren zur Herstellung von Verbindungen der vorstehenden Formel I gemäß Anspruch 1, dadurch gekennzeichnet, dass man Verbindungen der Formel II

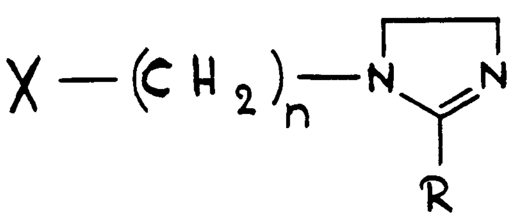

(II)

in der R, X und n die obige Bedeutung haben
mit Acrylsäure, Methacrylsäure, Maleinsäure oder aktiven Derivaten oder Mischungen davon in einem 2 bis 5fachen Ueberschuss kondensiert.

3. Reinigungsmittel enthaltend Verbindungen der vorstehenden Formel I gemäß Anspruch 1.

4. Reinigungs- und Pflegemittel enthaltend Mischungen von Verbindungen der Formel I gemäß Anspruch 1. und anionischen, kationischen, neutralen und/oder amphoteren Tensiden.

5. Reinigungs- und Pflegemittel gemäss Anspruch 4, wobei als anionische Tenside Fettalkoholsulfate, Fettalkoholethersulfate, Alkansulfonate,sekundäre Alkansulfonate enthalten sind.

6. Körperpflegemittel in Form von Duschgelen, Shampoos, Babyshampoos, Schaumbädern, Cremebädern, Oelbädern, Seifen, Flüssigseifen und kosmetischen Pflegeemulsionen, Gesichtswaschlotionen, dadurch gekennzeichnet, dass Abmischungen von anionischen, nichtionischen, kationischen und/oder amphoteren Tensiden zu Verbindungen der Formel I gemäß Anspruch 1 in einem Verhältnis von 2 : 98 bis 50 : 50, speziell 10 : 90 bis 20 : 80 enthalten.

7. Reinigungsmittel in Form von Handgeschirrspülmitteln, Allzweckreinigern, Feinwaschmitteln und Wollwaschmitteln, Maschinengeschirrspülmitteln, dadurch gekennzeichnet, dass man Abmischungen von kationischen, nichtionischen, anionischen und/oder amphoteren Tensiden und Verbindungen der Formel I gemäß Anspruch 1 im Verhältnis von 2 : 98 bis 20 : 80 verwendet.

## Claims

1. Polyimidazoline carbonic acid of the general formula I

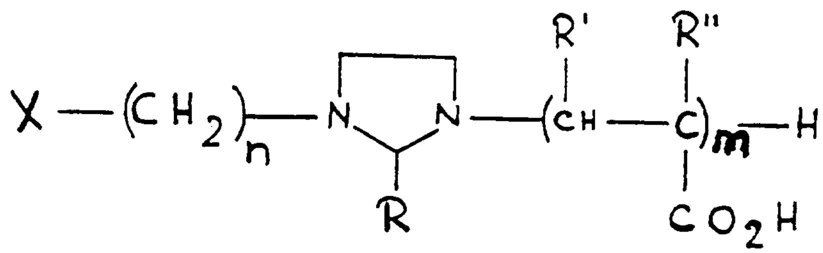

(I)

in which mean

| | |
|---|---|
| X = | -Cl, -OH, $-O-SO_3H$, $-O-PO_3H_2$, $-O-CH_2-CH_2-CO_2H$, $NH_2$, NHalk, $N(alk)_2$ with |
| alk = | lower alkyl with 1 to 6 carbon atoms, |
| n = | an integer from 2 to 4 |
| R = | a straight chained, branched, saturated or 1 to 3 times unsaturated hydrocarbon group with 8 to 18 C-atoms, |
| m = | an integer of 2 to 5 |
| R' = | a hydrogen radical or carboxylic group, |
| R'' = | a hydrogen radical or methyl group |

2. Process for the production of compounds of the above formula I according to claim I, characterised in condensing compounds of formula II

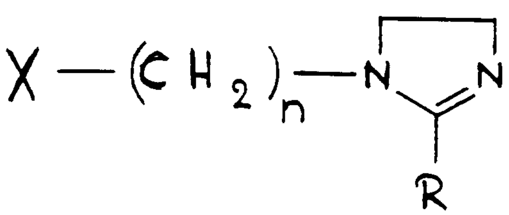

(II)

in which R, X have the above meaning,
with acrylic acid, methacrylic acid, malic acid or active derivatives or mixtures thereof in a 2 to 5 fold excess.

3. Cleaning agents comprising compounds of the above formula I according to claim 1.

4. Cleaning and caring agents comprising mixtures of compounds of formula I according to claim 1 and anionic, cationic, neutral and/or amphoteric tensides.

5. Cleaning and caring agents according to claim 4, wherein the anionic tensides comprise fatty alcohol sulfates, fatty alcohol ethersulfates, alkan sulfonates and secondary alkan sulfonates.

6. Body care agents in the form of shower gels, shampoos, baby shampoos, foam baths, cream baths, oil baths, soaps, liquid soaps and cosmetic care emulsions, face washing lotions characterised in comprising mixtures of anionic, nonionic, cationic and/or amphoteric tensides with compounds of formula I according to claim 1 in a ratio of 2:98 to 50:50, especially 10:90 to 20:80.

7. Cleaning agent in the form of manual dish washing agents, general use cleaning agents, mild detergents, wool detergents, mashine dish washing agents, characterised in using a mixture of cationic, nonionic, anionic and/or amphoteric tensides and compounds of formula I according to claim 1 in a ratio of 2:98 to 20:80.

## Revendications

1. Imidazolines d'acides polycarboxyliques répondant à la formule générale I

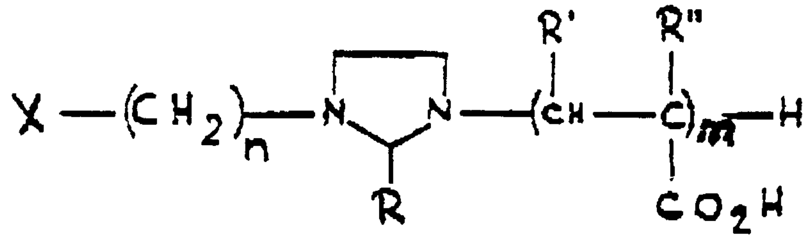

(I)

dans laquelle

X = -Cl, -OH, $-O-SO_3H$, $-O-PO_3H_2$ , $-O-CH_2-CH_2-CO_2H$, $NH_2$ , NHalk, $N(alk)_2$ où alk = alkyle inférieur comportant 1 à 5 atomes de C,

n = un nombre entier de 1 à 4,

R = un radical d'hydrocarbure linéaire, ramifié, saturé ou 1 à 3 fois insaturé, comportant 8 à 18 atomes de C,

m = un nombre entier de 2 à 5,

R' = un atome d'hydrogène ou un groupe carboxyle,

R'' = un atome d'hydrogène ou un groupe méthyle.

2. Procédé de préparation de composés de la formule I précédente selon la revendication 1, caractérisé en ce qu'on condense des composés de la formule II

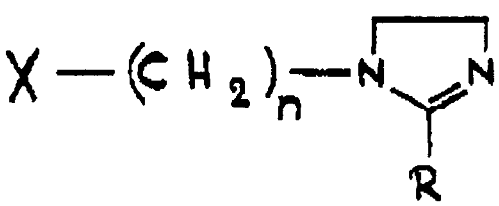

(II)

dans laquelle R, X et n ont la signification donnée ci-dessus, avec de l'acide acrylique, de l'acide méthacrylique, de l'acide maléique ou des dérivés actifs ou mélanges de ceux-ci, en un excès de 2 à 5 fois.

3. Produits de nettoyage contenant des composés répondant à la formule I précédente, suivant la revendication 1.

4. Produits de nettoyage et d'entretien contenant des mélanges de composés de la formule I selon la revendication 1 et des agents tensioactifs anioniques, cationiques, neutres et/ou amphotères.

5. Produits de nettoyage et d'entretien suivant la revendication 4, dans lesquels sont contenus, comme agents tensioactifs anioniques, des sulfates d'alcool gras, des éthersulfates d'alcool gras, des sulfonates d'alcane, des sulfonates d'alcane secondaire.

6. Produits d'entretien du corps sous la forme de gels de douche, de shampooings, de shampooings pour bébé, de bains mousse, de crèmes de bain, d'huiles de bain, de savons, de savons liquides et d'émulsions de soins cosmétiques, de lotions de lavage du visage, caractérisés en ce qu'ils contiennent des mélanges d'agents tensioactifs anioniques, non ionogènes, cationiques et/ou amphotères et des composés de la formule I selon la revendication 1 dans un rapport de 2/98 à 50/50, spécialement de 10/90 à 20/80.

**7.** Produits de nettoyage sous la forme de produits de lavage de vaisselle à la main, de produits de nettoyage à tout usage, de produits de lavage fin et de produits de lavage de laine, de produits de rinçage de vaisselle à la machine, caractérisés en ce que l'on utilise des mélanges d'agents tensioactifs cationiques, non ionogènes, anioniques et/ou amphotères et de composés de la formule I selon la revendication 1, dans un rapport de 2/98 à 20/80.